# EUROPEAN PATENT APPLICATION

(11) **EP 4 843 604 A1**
(43) Date of publication of application: **25.11.2026**
(21) Application number: 25741804.6
(22) Date of filing: 06.01.2025
(51) Int. Cl.: A61M 25/09

(54) **GUIDE WIRE AND CONNECTION STRUCTURE**

(30) Priority: 16.01.2024 JP 2024004243; 22.02.2024 JP 2024025042
(71) Applicant: ASAHI INTECC CO., LTD., Seto-shi, Aichi, 489-0071 (JP)
(72) Inventor: TAKAI HANAI, Ami, Seto-shi, Aichi 489-0071 (JP); ARAI, Yuki, Seto-shi, Aichi 489-0071 (JP)
(74) Representative: Winter, Brandl - Partnerschaft mbB
(86) International application number: PCT/JP2025/000032
(87) International publication number: WO 2025/154559

(57) **Abstract**

To provide a guide wire having distal end flexibility. A guide wire 1 includes a core shaft 9, a coil body 3 covering a distal end portion of the core shaft 9, a distal end connection portion 2 connecting a distal end of the coil body 3 and a distal end of the core shaft 9, and a proximal end connection portion 5 connecting a proximal end of the coil body 3 and the core shaft 9 and containing an adhesive made of a resin material.

## Description

### TECHNICAL FIELD

The present disclosure relates to a medical guide wire and a connection structure.

### BACKGROUND ART

Patent Literature 1 describes a guide wire including a core wire, a metal coil mounted on the outer periphery of a distal end portion of the core wire, a brazed portion mounted between a distal end portion of the metal coil and the distal end portion of the core wire, a brazed portion mounted between an intermediate portion of the metal coil and the core wire, and a brazed portion mounted between a proximal end portion of the metal coil and the core wire.

### CITATION LIST

### Patent Literature

Patent Literature 1: JP 2011-143077 A

### SUMMARY OF INVENTION

### TECHNICAL PROBLEM

In the guide wire described in Patent Literature 1, since the brazed portion is composed of a metal material, there is a problem that the rigidity of the guide wire becomes high, particularly at the brazed portion.

Since a guide wire is inserted along a meandering blood vessel in a patient's body, the guide wire may become stuck in the patient's body during a procedure, and there is a risk that the metal coil (hereinafter referred to as "coil body") may become detached from the core wire (hereinafter referred to as "core shaft") due to excessive force applied by an operator thereafter. Therefore, assuming such a situation, it is desirable to take safety measures in advance.

The present embodiments have been made in view of the above-described problems, and an object thereof is to provide a guide wire having distal end flexibility.

### SOLUTION TO PROBLEM

In order to solve the above-described problem, a guide wire according to a first aspect includes a core shaft, a coil body covering a distal end portion of the core shaft, a distal end connection portion connecting a distal end of the coil body and a distal end of the core shaft, and a proximal end connection portion connecting a proximal end of the coil body and the core shaft and containing an adhesive made of a resin material.

According to the guide wire of the first aspect, the guide wire can be made flexible. Therefore, the insertability of the guide wire into the patient's body can be improved.

A guide wire according to a second aspect is the guide wire according to the first aspect, in which the coil body is wound a plurality of times around an outer periphery of the core shaft, and the proximal end connection portion may be formed to have an outer diameter smaller than an outer diameter of the coil body between adjacent strands of the coil body.

According to the guide wire of the second aspect, the guide wire can be made even more flexible. Therefore, the insertability of the guide wire into the patient's body can be further improved.

A guide wire according to a third aspect is the guide wire according to the first or second aspect, in which the proximal end connection portion may be colored.

According to the guide wire of the third aspect, the length of the proximal end connection portion connected to the coil body can be easily grasped. Therefore, the joint strength between the core shaft and the coil body can be easily adjusted.

A guide wire according to a fourth aspect is the guide wire according to any one of the first to third aspects, including an intermediate connection portion connecting the coil body and the core shaft between the distal end connection portion and the proximal end connection portion, in which the intermediate connection portion may contain an adhesive made of a resin material.

According to the guide wire of the fourth aspect, the coil body and the core shaft can be connected at an intermediate position between the distal end and the proximal end of the coil body, and the guide wire can be made flexible. Therefore, the insertability of the guide wire into the patient's body can be improved.

A guide wire according to a fifth aspect is the guide wire according to the fourth aspect, in which the coil body is wound a plurality of times around the outer periphery of the core shaft, and the intermediate connection portion may be formed to have an outer diameter smaller than the outer diameter of the coil body between adjacent strands of the coil body.

According to the guide wire of the fifth aspect, the guide wire can be made even more flexible. Therefore, the insertability of the guide wire into the patient's body can be further improved.

A guide wire according to a sixth aspect is the guide wire according to the fourth or fifth aspect, in which the intermediate connection portion may be colored.

According to the guide wire of the sixth aspect, the length of the intermediate connection portion connected to the coil body can be easily grasped. Therefore, the joint strength between the core shaft and the coil body can be easily adjusted.

A guide wire according to a seventh aspect is the guide wire according to any one of the first to sixth aspects, in which a surface roughness of the coil body may be greater than a surface roughness of the core shaft.

According to the guide wire of the seventh aspect, even in a case where a large force is applied to the guide wire and the coil body is detached from the core shaft, the proximal end connection portion is detached in a state of being connected to the coil body, so that the proximal end connection portion can be prevented from being crushed and scattered.

A guide wire according to an eighth aspect is the guide wire according to any one of the first to seventh aspects, in which an oxide film may be formed on a surface of the coil body.

According to the guide wire of the eighth aspect, even in a case where a large force is applied to the guide wire and the coil body is detached from the core shaft, the proximal end connection portion is detached in a state of being connected to the coil body, so that the proximal end connection portion can be prevented from being crushed and scattered.

A guide wire according to a ninth aspect is the guide wire according to any one of the first to eighth aspects, in which a plurality of grooves may be formed on the surface of the coil body in a direction intersecting a longitudinal direction of the core shaft.

According to the guide wire of the ninth aspect, even in a case where a large force is applied to the guide wire and the coil body is detached from the core shaft, the proximal end connection portion is detached in a state of being connected to the coil body, so that the proximal end connection portion can be prevented from being crushed and scattered.

A guide wire according to a tenth aspect is the guide wire according to any one of the first to ninth aspects, in which a plurality of grooves may be formed on a surface of the core shaft in a direction intersecting the longitudinal direction of the core shaft.

According to the guide wire of the tenth aspect, even in a case where a large force is applied to the guide wire and the coil body is detached from the core shaft, the proximal end connection portion is detached in a state of being connected to the coil body, so that the proximal end connection portion can be prevented from being crushed and scattered.

A guide wire according to an eleventh aspect is the guide wire according to any one of the first to ninth aspects, in which a plurality of grooves may be formed on the surface of the core shaft along the longitudinal direction of the core shaft.

According to the guide wire of the eleventh aspect, even in a case where a large force is applied to the guide wire and the coil body is detached from the core shaft, the proximal end connection portion is detached in a state of being connected to the core shaft, so that the proximal end connection portion can be prevented from being crushed and scattered.

A connection structure according to a twelfth aspect connects a surface of a first member having a first surface roughness and a surface of a second member having a second surface roughness greater than the first surface roughness by an adhesive made of a resin material.

According to the connection structure of the twelfth aspect, even in a case where a large force is applied to the connection structure and the second member is detached from the first member, the adhesive is detached in a state of being connected to the second member, so that the adhesive can be prevented from being crushed and scattered.

A connection structure according to a thirteenth aspect connects a surface of a first member on which no oxide film is formed and a surface of a second member on which an oxide film is formed by an adhesive made of a resin material.

According to the connection structure of the thirteenth aspect, even in a case where a large force is applied to the connection structure and the second member is detached from the first member, the adhesive is detached in a state of being connected to the second member, so that the adhesive can be prevented from being crushed and scattered.

### BRIEF DESCRIPTION OF DRAWINGS

FIG. 1 is an overall schematic view of a guide wire of a first embodiment.
FIG. 2 is an enlarged view of a distal end of the guide wire of the first embodiment.
FIG. 3 is a longitudinal sectional view of the distal end of the guide wire of the first embodiment.
FIG. 4 is a longitudinal sectional view of a distal end of a guide wire of a second embodiment.
FIG. 5 is a longitudinal sectional view of a distal end of a guide wire of a third embodiment.
FIG. 6 is a longitudinal sectional view of a distal end of a guide wire of a fourth embodiment.
FIG. 7 is a longitudinal sectional view of a distal end of a guide wire of a fifth embodiment.
FIG. 8 is a longitudinal sectional view of a distal end of a guide wire of a sixth embodiment.
FIG. 9 is an enlarged view of part A of FIG. 8.
FIG. 10 is an enlarged view of the distal end showing a state in which a coil body of the guide wire of the sixth embodiment is detached from a core shaft.
FIG. 11 is a longitudinal sectional view of a distal end of a guide wire of a seventh embodiment.
FIG. 12 is an enlarged view of part B of FIG. 11.
FIG. 13 is an enlarged view of a distal end of a guide wire of an eighth embodiment.
FIG. 14 is a partially enlarged view of a guide wire of a ninth embodiment.
FIG. 15 is a partially enlarged view of a guide wire of a tenth embodiment.

### DESCRIPTION OF EMBODIMENTS

Hereinafter, several present embodiments will be described with reference to the drawings. The drawings used in the following embodiments are exaggerated for easy understanding, and their dimensions are different from actual dimensions. Portions common to the respective embodiments are denoted by the same reference symbols, and descriptions of portions already described may be omitted.

### (First Embodiment)

A first embodiment of the present disclosure will be described.

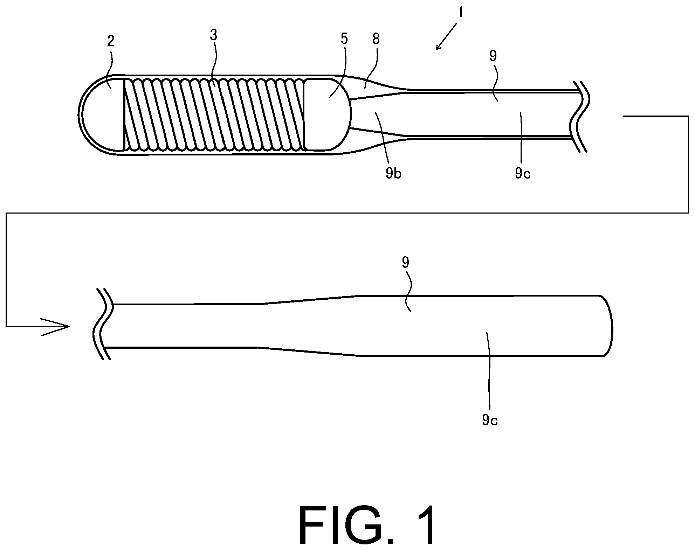

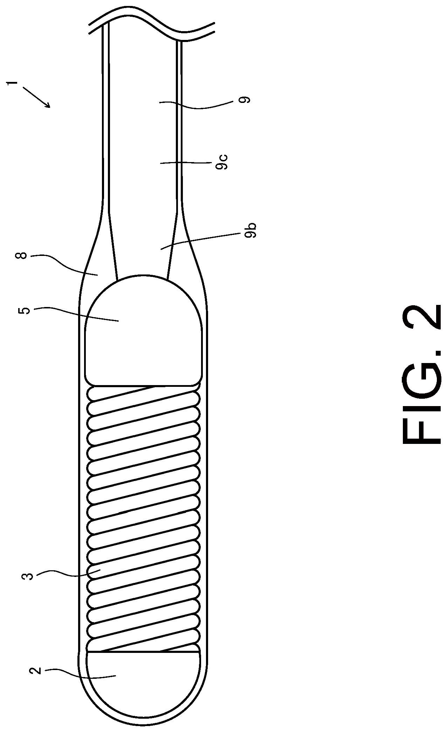

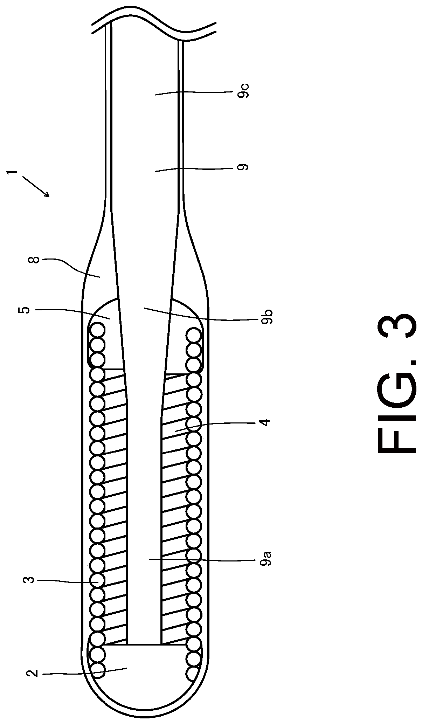

FIG. 1 is an overall schematic view of the guide wire of the first embodiment, FIG. 2 is an enlarged view of the distal end of the guide wire of the first embodiment, and FIG. 3 is a longitudinal sectional view of the distal end of the guide wire of the first embodiment.

As shown in FIGS. 1 to 3, a guide wire 1 of the present embodiment includes a core shaft 9, a coil body 3 covering a distal end portion of the core shaft 9, a distal end connection portion 2 connected to a distal end of the coil body 3 and a distal end of the core shaft 9, a proximal end connection portion 5 connected to a proximal end of the coil body 3 and the core shaft 9, and a coating agent 8 covering outer peripheries of the core shaft 9, the coil body 3, the distal end connection portion 2, and the proximal end connection portion 5.

The core shaft 9 is an elongated flexible member having a substantially circular cross section and decreasing in diameter from the proximal end toward the distal end. The core shaft 9 includes a proximal end side cylindrical portion 9c having a substantially cylindrical shape as a whole, a distal end-side tapered portion 9b formed continuously from a distal end of the proximal end side cylindrical portion 9c toward a distal end side and gradually decreasing in diameter toward the distal end, and a distal end-side cylindrical portion 9a formed continuously from a distal end of the distal end-side tapered portion 9b toward the distal end side and having a substantially cylindrical shape.

The material of the core shaft 9 is not particularly limited as long as it is a biocompatible material, such as metal materials such as stainless steel, Ni-Ti alloys, and cobalt alloys, or synthetic resin materials such as polyethylene, polyethylene terephthalate, polypropylene, polyurethane, and polyvinyl chloride. In the present embodiment, the material of the core shaft 9 is, for example, stainless steel.

The coil body 3 is a hollow cylindrical coil body formed by winding either of one or more metal strands or one or more resin strands. The distal end of the coil body 3 is connected to the distal end of the core shaft 9 by the distal end connection portion 2, and the proximal end of the coil body 3 is connected to the core shaft 9 by the proximal end connection portion 5.

The material of the strand forming the coil body 3 is not particularly limited as long as it is a biocompatible material, such as metal materials such as stainless steel, Ni-Ti alloys, and cobalt alloys, or synthetic resin materials such as polyethylene, polyethylene terephthalate, polypropylene, polyurethane, and polyvinyl chloride. In the present embodiment, the material of the strand is stainless steel.

As shown in FIG. 3, a gap 4 is formed between the core shaft 9 and the coil body 3.

The distal end connection portion 2 joins the distal end of the coil body 3 and the distal end of the core shaft 9. The material of the distal end connection portion 2 is not particularly limited as long as it is a biocompatible joining member such as a gold-tin solder or a silver-tin solder. In the present embodiment, the material of the distal end connection portion 2 is a silver-tin solder.

The proximal end connection portion 5 joins the proximal end of the coil body 3 and the core shaft 9. The material of the proximal end connection portion 5 is not particularly limited as long as it is a biocompatible joining member made of a resin material, such as a silicone-based adhesive, an acrylic-based adhesive, an epoxy-based adhesive, or a cyanoacrylate-based adhesive. In the present embodiment, the material of the proximal end connection portion 5 is an epoxy-based adhesive.

The coating agent 8 covers the outer peripheries of the core shaft 9, the coil body 3, the distal end connection portion 2, and the proximal end connection portion 5. As the material of the coating agent 8, it is desirable to use, for example, polyvinyl alcohol, polyvinylpyrrolidone, polyethylene glycol, polyacrylamide, polyacrylic acid, sodium polyacrylate, poly(2-hydroxyethyl methacrylate), maleic anhydride-based copolymers, ethylene vinyl alcohol copolymers, 2-methacryloyloxyethyl phosphorylcholine, copolymers of 2-methacryloyloxyethyl phosphorylcholine, (2-hydroxyethyl methacrylate)-styrene block copolymers, various synthetic polypeptides, collagen, hyaluronic acid, cellulosic polymers, and mixtures thereof. In the present embodiment, the material of the coating agent 8 is polyacrylamide.

In the present embodiment, it is described that an adhesive made of a resin material is used only for the proximal end connection portion 5. An adhesive made of a resin material similar to that of the proximal end connection portion 5 may also be used for the distal end connection portion 2, provided that sufficient joint strength is secured.

The point that an adhesive made of a resin material similar to that of the proximal end connection portion 5 can be used for the distal end connection portion 2 applies not only to the present embodiment but also to the embodiments described later.

According to the guide wire 1 of the present embodiment, the guide wire 1 includes the core shaft 9, the coil body 3 covering the distal end portion of the core shaft 9, the distal end connection portion 2 connecting the distal end of the coil body 3 and the distal end of the core shaft 9, and the proximal end connection portion 5 connecting the proximal end of the coil body 3 and the core shaft 9, and since the proximal end connection portion 5 is composed of an adhesive made of a resin material, the guide wire 1 can be made flexible. Therefore, the insertability of the guide wire 1 into the patient's body can be improved.

### (Second Embodiment)

A second embodiment will be described.

A guide wire 10 of the second embodiment differs from the guide wire 1 of the first embodiment in the number of connection portions connecting the coil body and the core shaft. That is, the guide wire 1 of the first embodiment was provided with two connection portions: the distal end connection portion 2 connecting the distal end of the coil body 3 and the distal end of the core shaft 9, and the proximal end connection portion 5 connecting the proximal end of the coil body 3 and the core shaft 9. The guide wire 10 of the present embodiment includes, in addition to the distal end connection portion and the proximal end connection portion, intermediate connection portions arranged between the distal end connection portion and the proximal end connection portion in the longitudinal direction of the guide wire and connecting the coil body and the core shaft.

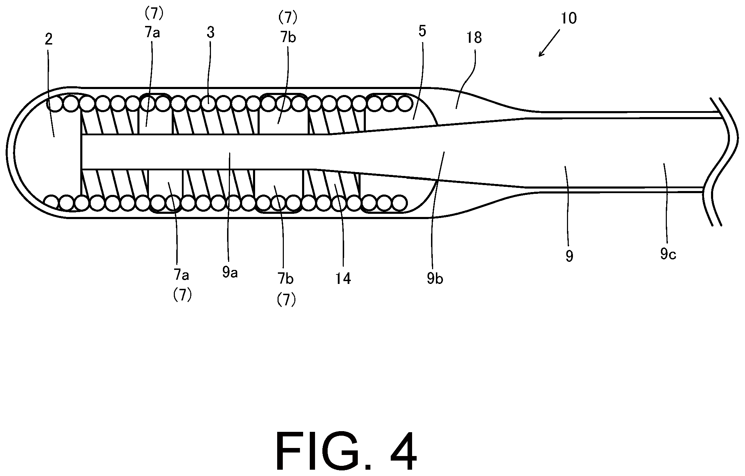

FIG. 4 is a longitudinal sectional view of a distal end of the guide wire of the second embodiment.

The guide wire 10 of the present embodiment includes a core shaft 9, a coil body 3 covering a distal end portion of the core shaft 9, a distal end connection portion 2 connected to a distal end of the coil body 3 and a distal end of the core shaft 9, a proximal end connection portion 5 connected to a proximal end of the coil body 3 and the core shaft 9, a plurality of intermediate connection portions 7 arranged between the distal end connection portion 2 and the proximal end connection portion 5 in the longitudinal direction of the guide wire 10 and connected to the coil body 3 and the core shaft 9, namely, a distal end side intermediate connection portion 7a and a proximal end side intermediate connection portion 7b, and a coating agent 18 covering outer peripheries of the core shaft 9, the coil body 3, the distal end connection portion 2, the proximal end connection portion 5, the distal end side intermediate connection portion 7a, and the proximal end side intermediate connection portion 7b.

A gap 14 is formed between the core shaft 9 and the coil body 3.

The distal end side intermediate connection portion 7a is arranged between the distal end connection portion 2 and the proximal end connection portion 5 in the longitudinal direction of the guide wire 10 and connects the coil body 3 and the core shaft 9. The material of the distal end side intermediate connection portion 7a is not particularly limited as long as it is a biocompatible joining member made of a resin material, such as a silicone-based adhesive, an acrylic-based adhesive, an epoxy-based adhesive, or a cyanoacrylate-based adhesive. In the present embodiment, the material of the distal end side intermediate connection portion 7a is an epoxy-based adhesive.

The proximal end side intermediate connection portion 7b is arranged between the distal end connection portion 2 and the proximal end connection portion 5 in the longitudinal direction of the guide wire 10 and connects the coil body 3 and the core shaft 9 on a more proximal end side than the distal end side intermediate connection portion 7a. The material of the proximal end side intermediate connection portion 7b, such as the proximal end connection portion 5, is not particularly limited as long as it is a biocompatible joining member made of a resin material, such as a silicone-based adhesive, an acrylic-based adhesive, an epoxy-based adhesive, or a cyanoacrylate-based adhesive. In the present embodiment, the material of the proximal end side intermediate connection portion 7b is an epoxy-based adhesive.

In the present embodiment, as shown in FIG. 4, two intermediate connection portions, the distal end side intermediate connection portion 7a and the proximal end side intermediate connection portion 7b, are provided. The number of intermediate connection portions is not limited to two, and may be one, or three or more.

The coating agent 18 covers the outer peripheries of the core shaft 9, the coil body 3, the distal end connection portion 2, the proximal end connection portion 5, the distal end side intermediate connection portion 7a, and the proximal end side intermediate connection portion 7b. The material of the coating agent 18 may be the same as that of the coating agent 8 of the first embodiment, and in the present embodiment, is polyacrylamide.

According to the guide wire 10 of the present embodiment, the guide wire 10 includes the core shaft 9, the coil body 3 covering the distal end portion of the core shaft 9, the distal end connection portion 2 connecting the distal end of the coil body 3 and the distal end of the core shaft 9, and the proximal end connection portion 5 connecting the proximal end of the coil body 3 and the core shaft 9, and since the proximal end connection portion 5 is composed of an adhesive made of a resin material, the guide wire 10 can be made flexible. Therefore, the insertability of the guide wire 10 into the patient's body can be improved.

According to the guide wire 10 of the present embodiment, the intermediate connection portion 7 connecting the coil body 3 and the core shaft 9 is provided between the distal end connection portion 2 and the proximal end connection portion 5, and since the intermediate connection portion 7 is composed of an adhesive made of a resin material, the guide wire 10 can be made flexible. Therefore, the insertability of the guide wire 10 into the patient's body can be improved.

### (Third Embodiment)

A third embodiment will be described.

A guide wire 20 of the third embodiment differs from the guide wire 1 of the first embodiment in the form of the proximal end connection portion. That is, while the outer diameter of the proximal end connection portion 5 in the guide wire 1 of the first embodiment was substantially the same between the strands constituting the coil body 3, the outer diameter of the proximal end connection portion in the guide wire 20 of the present embodiment is smaller than the outer diameter of the coil body between the strands constituting the coil body.

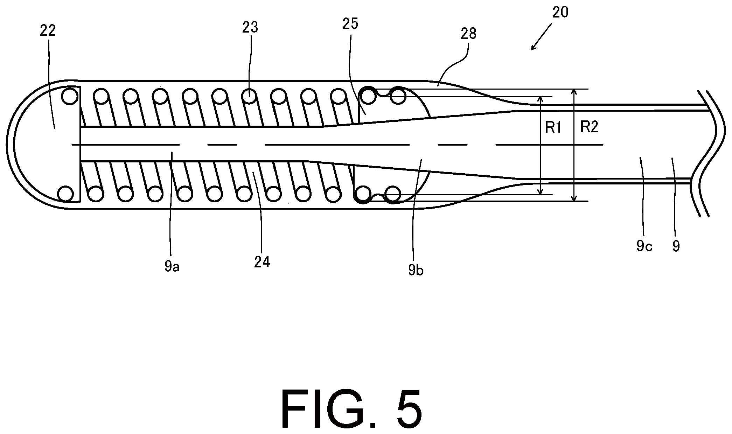

FIG. 5 is a longitudinal sectional view of a distal end of the guide wire of the third embodiment.

The guide wire 20 of the present embodiment includes a core shaft 9, a coil body 23 covering a distal end portion of the core shaft 9, a distal end connection portion 22 connected to a distal end of the coil body 23 and a distal end of the core shaft 9, a proximal end connection portion 25 connected to a proximal end of the coil body 23 and the core shaft 9, and a coating agent 28 covering outer peripheries of the core shaft 9, the coil body 23, the distal end connection portion 22, and the proximal end connection portion 25.

A gap 24 is formed between the core shaft 9 and the coil body 23.

The coil body 23 is a hollow cylindrical coil body formed by winding either of one or more metal strands or one or more resin strands. The coil body 23 is wound a plurality of times around the outer periphery of the core shaft 9. The distal end of the coil body 23 is connected to the distal end of the core shaft 9 by the distal end connection portion 22, and the proximal end of the coil body 23 is connected to the core shaft 9 by the proximal end connection portion 25.

The material of the strand forming the coil body 23 may be the same as that of the coil body 3 of the first embodiment. In the present embodiment, the material of the strand forming the coil body 23 is stainless steel.

The distal end connection portion 22 joins the distal end of the coil body 23 and the distal end of the core shaft 9. The material of the distal end connection portion 22 may be the same as that of the distal end connection portion 2 of the first embodiment. In the present embodiment, the material of the distal end connection portion 22 is a silver-tin solder.

The proximal end connection portion 25 joins the proximal end of the coil body 23 and the core shaft 9. An outer diameter R1 of the proximal end connection portion 25 is smaller than an outer diameter R2 of the coil body 23 between the strands constituting the coil body 23.

The material of the proximal end connection portion 25 may be the same as that of the proximal end connection portion 5 of the first embodiment. In the present embodiment, the material of the proximal end connection portion 25 is an epoxy-based adhesive.

In the present embodiment, the longitudinal width of the proximal end connection portion 25 is formed across two strands constituting the coil body 23, and one portion having the outer diameter R1 smaller than the outer diameter R2 of the coil body 23 is provided in the proximal end connection portion 25. The longitudinal width of the proximal end connection portion 25 may be formed across any number of strands of the coil body 23. A plurality of portions having the outer diameter R1 smaller than the outer diameter R2 of the coil body 23 may be formed in the proximal end connection portion 25.

The coating agent 28 covers the outer peripheries of the core shaft 9, the coil body 23, the distal end connection portion 22, and the proximal end connection portion 25. The material of the coating agent 28 may be the same as that of the coating agent 8 of the first embodiment. In the present embodiment, the material of the coating agent 28 is polyacrylamide.

According to the guide wire 20 of the present embodiment, the guide wire 20 includes the core shaft 9, the coil body 23 covering the distal end portion of the core shaft 9, the distal end connection portion 22 connecting the distal end of the coil body 23 and the distal end of the core shaft 9, and the proximal end connection portion 25 connecting the proximal end of the coil body 23 and the core shaft 9, and since the proximal end connection portion 25 is composed of an adhesive made of a resin material, the guide wire 20 can be made flexible. Therefore, the insertability of the guide wire 20 into the patient's body can be improved.

According to the guide wire 20 of the present embodiment, the coil body 23 is wound a plurality of times around the outer periphery of the core shaft 9, and since the proximal end connection portion 25 is formed to have an outer diameter smaller than the outer diameter of the coil body 23 between adjacent strands constituting the coil body 23, the guide wire 20 can be made even more flexible. Therefore, the insertability of the guide wire 20 into the patient's body can be further improved.

### (Fourth Embodiment)

A fourth embodiment will be described.

A guide wire 30 of the fourth embodiment differs from the guide wire 20 of the third embodiment in the number of connection portions connecting the coil body and the core shaft. That is, the guide wire 20 of the third embodiment was provided with two connection portions: the distal end connection portion 22 connecting the distal end of the coil body 23 and the distal end of the core shaft 9, and the proximal end connection portion 25 connecting the proximal end of the coil body 23 and the core shaft 9. The guide wire 30 of the present embodiment includes, in addition to the distal end connection portion and the proximal end connection portion, an intermediate connection portion arranged between the distal end connection portion and the proximal end connection portion in the longitudinal direction of the guide wire and connecting the coil body and the core shaft.

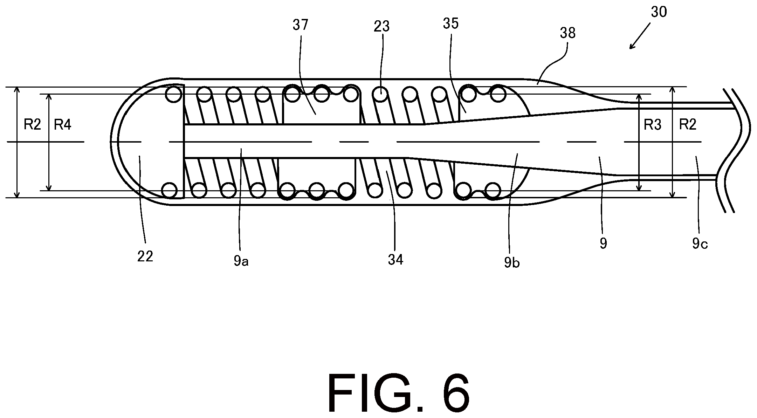

FIG. 6 is a longitudinal sectional view of a distal end of the guide wire of the fourth embodiment.

The guide wire 30 of the present embodiment includes a core shaft 9, a coil body 23 covering a distal end portion of the core shaft 9, a distal end connection portion 22 connected to a distal end of the coil body 23 and a distal end of the core shaft 9, a proximal end connection portion 35 connected to a proximal end of the coil body 23 and the core shaft 9, an intermediate connection portion 37 arranged between the distal end connection portion 22 and the proximal end connection portion 35 in the longitudinal direction of the guide wire 30 and connected to the coil body 23 and the core shaft 9, and a coating agent 38 covering outer peripheries of the core shaft 9, the coil body 23, the distal end connection portion 22, the proximal end connection portion 35, and the intermediate connection portion 37.

A gap 34 is formed between the core shaft 9 and the coil body 23.

The proximal end connection portion 35 joins the proximal end of the coil body 23 and the core shaft 9. An outer diameter R3 of the proximal end connection portion 35 is smaller than the outer diameter R2 of the coil body 23 between the strands constituting the coil body 23.

The material of the proximal end connection portion 35 may be the same as that of the proximal end connection portion 5 of the first embodiment. In the present embodiment, the material of the proximal end connection portion 35 is an epoxy-based adhesive.

The intermediate connection portion 37 is arranged between the distal end connection portion 22 and the proximal end connection portion 35 in the longitudinal direction of the guide wire 30 and connects the coil body 23 and the core shaft 9. An outer diameter R4 of the intermediate connection portion 37 is smaller than the outer diameter R2 of the coil body 23 between the strands constituting the coil body 23.

The material of the intermediate connection portion 37 may be the same as that of the distal end side intermediate connection portion 7a and the proximal end side intermediate connection portion 7b of the second embodiment. In the present embodiment, the material of the intermediate connection portion 37 is an epoxy-based adhesive.

In the present embodiment, one intermediate connection portion 37 is provided. The number of intermediate connection portions 37 is not limited to one, and may be two or more.

In the present embodiment, the longitudinal width of the proximal end connection portion 35 is formed across two strands constituting the coil body 23, and one portion having the outer diameter R3 smaller than the outer diameter R2 of the coil body 23 is provided in the proximal end connection portion 35. The longitudinal width of the proximal end connection portion 35 may be formed across any number of strands of the coil body 23. A plurality of portions having the outer diameter R3 smaller than the outer diameter R2 of the coil body 23 may be formed in the proximal end connection portion 35.

In the present embodiment, the longitudinal width of the intermediate connection portion 37 is formed across three strands constituting the coil body 23, and two portions having the outer diameter R4 smaller than the outer diameter R2 of the coil body 23 are provided in the intermediate connection portion 37. The longitudinal width of the intermediate connection portion 37 may be formed across any number of strands of the coil body 23. The number of portions having the outer diameter R4 smaller than the outer diameter R2 of the coil body 23 may be one, or three or more in the intermediate connection portion 37.

The coating agent 38 covers the outer peripheries of the core shaft 9, the coil body 23, the distal end connection portion 22, the proximal end connection portion 35, and the intermediate connection portion 37. The material of the coating agent 38 may be the same as that of the coating agent 8 of the first embodiment. In the present embodiment, the material of the coating agent 38 is polyacrylamide.

According to the guide wire 30 of the present embodiment, the guide wire 30 includes the core shaft 9, the coil body 23 covering the distal end portion of the core shaft 9, the distal end connection portion 22 connecting the distal end of the coil body 23 and the distal end of the core shaft 9, and the proximal end connection portion 35 connecting the proximal end of the coil body 23 and the core shaft 9, and since the proximal end connection portion 35 is composed of an adhesive made of a resin material, the guide wire 30 can be made flexible. Therefore, the insertability of the guide wire 30 into the patient's body can be improved.

According to the guide wire 30 of the present embodiment, the intermediate connection portion 37 connecting the coil body 23 and the core shaft 9 is provided between the distal end connection portion 22 and the proximal end connection portion 35, and since the intermediate connection portion 7 is composed of an adhesive made of a resin material, the guide wire 30 can be made flexible. Therefore, the insertability of the guide wire 30 into the patient's body can be improved.

According to the guide wire 30 of the present embodiment, the coil body 23 is wound a plurality of times around the outer periphery of the core shaft 9, and since the proximal end connection portion 35 is formed to have an outer diameter smaller than the outer diameter of the coil body 23 between adjacent strands constituting the coil body 23, the guide wire 30 can be made even more flexible. Therefore, the insertability of the guide wire 30 into the patient's body can be further improved.

Furthermore, according to the guide wire 30 of the present embodiment, the coil body 23 is wound a plurality of times around the outer periphery of the core shaft 9, and since at least one intermediate connection portion 37 is formed at a position lower than the outer diameter of the coil body 23 between adjacent strands constituting the coil body 23, the guide wire 30 can be made even more flexible, and consequently, the insertability of the guide wire 30 into the patient's body can be further improved.

### (Fifth Embodiment)

A fifth embodiment will be described.

A guide wire 40 of the fifth embodiment differs from the guide wire 30 of the fourth embodiment in the color of the proximal end connection portion and the intermediate connection portion. That is, while the colors of the proximal end connection portion 35 and the intermediate connection portion 37 of the guide wire 30 of the fourth embodiment are colorless and transparent, the colors of the proximal end connection portion and the intermediate connection portion of the guide wire 40 of the present embodiment are colored.

In the present embodiment, "colored" means a magenta color, a cyan color, a yellow color, and a black color, as well as colors formed by combining these colors.

By coloring the proximal end connection portion, the length of the proximal end connection portion connected to the coil body can be easily grasped, and the joint strength between the core shaft and the coil body can be easily adjusted. By coloring the intermediate connection portion, the length of the intermediate connection portion connected to the coil body can be easily grasped, and the joint strength between the core shaft and the coil body can be easily adjusted.

This is based on the theory that since the winding pitch of the coil body with respect to the core shaft is known in advance when manufacturing the guide wire, by adjusting the guide wire's longitudinal lengths of the proximal end connection portion and the intermediate connection portion connected to the coil body, the number of strands of the coil body accommodated in the proximal end connection portion and the intermediate connection portion can be adjusted, thereby adjusting the joint strength between the core shaft and the coil body. Accordingly, if the length of the intermediate connection portion connected to the coil body can be easily grasped, the joint strength between the core shaft and the coil body can be easily grasped.

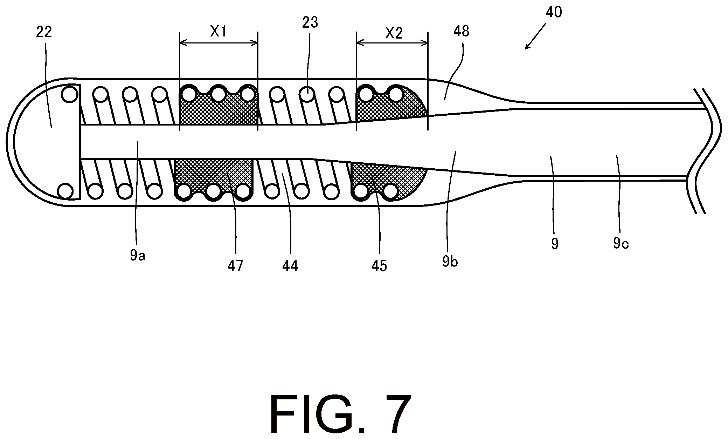

FIG. 7 is a longitudinal sectional view of a distal end of the guide wire of the fifth embodiment. In FIG. 7, colored portions are indicated by mesh-like hatching.

The guide wire 40 of the present embodiment includes a core shaft 9, a coil body 23 covering a distal end portion of the core shaft 9, a distal end connection portion 22 connected to a distal end of the coil body 23 and a distal end of the core shaft 9, a proximal end connection portion 45 connected to a proximal end of the coil body 23 and the core shaft 9, an intermediate connection portion 47 arranged between the distal end connection portion 22 and the proximal end connection portion 45 in the longitudinal direction of the guide wire 40 and connected to the coil body 23 and the core shaft 9, and a coating agent 48 covering outer peripheries of the core shaft 9, the coil body 23, the distal end connection portion 22, the proximal end connection portion 45, and the intermediate connection portion 47.

A gap 44 is formed between the core shaft 9 and the coil body 23.

The proximal end connection portion 45 joins the proximal end of the coil body 23 and the core shaft 9. The outer diameter of the proximal end connection portion 45 is smaller than the outer diameter of the coil body 23 between the strands constituting the coil body 23.

As the material of the proximal end connection portion 45, a material in which a pigment is contained in a material similar to that of the proximal end connection portion 5 of the first embodiment can be used. In the present embodiment, as the material of the proximal end connection portion 45, an epoxy-based adhesive containing a biocompatible ink mainly composed of hydroxypropyl cellulose (HPC) and gelatin is used. As another aspect, the material of the proximal end connection portion 45 may contain a component that emits light when irradiated with ultraviolet rays. In this case, the proximal end connection portion 45 may be colorless or colored under visible light. Such a component may be, for example, an epoxy resin, an acrylic resin, or the like, which is a resin having an absorption peak at the wavelength of ultraviolet rays.

The intermediate connection portion 47 is arranged between the distal end connection portion 22 and the proximal end connection portion 45 in the longitudinal direction of the guide wire 40 and connects the coil body 23 and the core shaft 9. The outer diameter of the intermediate connection portion 47 is smaller than the outer diameter of the coil body 23 between the strands constituting the coil body 23.

As the material of the intermediate connection portion 47, a material in which a pigment is contained in a material similar to those of the distal end side intermediate connection portion 7a and the proximal end side intermediate connection portion 7b of the second embodiment can be used. In the present embodiment, such as the material of the proximal end connection portion 45, an epoxy-based adhesive containing a biocompatible ink mainly composed of hydroxypropyl cellulose (HPC) and gelatin is used as the material of the intermediate connection portion 47. As another aspect, the material of the intermediate connection portion 47 may contain a component that emits light when irradiated with ultraviolet rays. In this case, the intermediate connection portion 47 may be colorless or colored under visible light. Such a component may be, for example, an epoxy resin, an acrylic resin, or the like, which is a resin having an absorption peak at the wavelength of ultraviolet rays.

In the present embodiment, one intermediate connection portion 47 is provided. The number of intermediate connection portions 47 is not limited to one, and may be two or more.

In the present embodiment, the longitudinal width of the proximal end connection portion 35 is formed across two strands constituting the coil body 23, and one portion having an outer diameter smaller than the outer diameter of the coil body 23 is provided in the proximal end connection portion 45. The longitudinal width of the proximal end connection portion 45 may be formed across any number of strands of the coil body 23. A plurality of portions having an outer diameter smaller than the outer diameter of the coil body 23 may be formed in the proximal end connection portion 45.

The longitudinal width of the intermediate connection portion 47 is formed across three strands constituting the coil body 23, and two portions having an outer diameter smaller than the outer diameter of the coil body 23 are provided in the intermediate connection portion 47. The longitudinal width of the intermediate connection portion 47 may be formed across any number of strands of the coil body 23. The number of portions having an outer diameter smaller than the outer diameter of the coil body 23 may be one, or three or more in the intermediate connection portion 47.

The coating agent 48 covers the outer peripheries of the core shaft 9, the coil body 23, the distal end connection portion 22, the proximal end connection portion 45, and the intermediate connection portion 47. The material of the coating agent 48 may be the same as that of the coating agent 8 of the first embodiment. In the present embodiment, the material of the coating agent 48 is polyacrylamide.

According to the guide wire 40 of the present embodiment, the guide wire 40 includes the core shaft 9, the coil body 23 covering the distal end portion of the core shaft 9, the distal end connection portion 22 connecting the distal end of the coil body 23 and the distal end of the core shaft 9, and the proximal end connection portion 45 connecting the proximal end of the coil body 23 and the core shaft 9, and since the proximal end connection portion 45 is composed of an adhesive made of a resin material, the guide wire 40 can be made flexible. Therefore, the insertability of the guide wire 40 into the patient's body can be improved.

According to the guide wire 40 of the present embodiment, the intermediate connection portion 47 connecting the coil body 23 and the core shaft 9 is provided between the distal end connection portion 22 and the proximal end connection portion 45, and since the intermediate connection portion 47 is composed of an adhesive made of a resin material, the guide wire 40 can be made flexible. Therefore, the insertability of the guide wire 40 into the patient's body can be improved.

According to the guide wire 40 of the present embodiment, the coil body 23 is wound a plurality of times around the outer periphery of the core shaft 9, and since the proximal end connection portion 45 is formed to have an outer diameter smaller than the outer diameter of the coil body 23 between adjacent strands constituting the coil body 23, the guide wire 40 can be made even more flexible. Therefore, the insertability of the guide wire 40 into the patient's body can be further improved.

According to the guide wire 40 of the present embodiment, the coil body 23 is wound a plurality of times around the outer periphery of the core shaft 9, and since the intermediate connection portion 47 is formed to have an outer diameter smaller than the outer diameter of the coil body 23 between adjacent strands constituting the coil body 23, the guide wire 40 can be made even more flexible. Therefore, the insertability of the guide wire 40 into the patient's body can be further improved.

According to the guide wire 40 of the present embodiment, since the proximal end connection portion 45 is colored, a length X2 of the proximal end connection portion 45 connected to the coil body 23 in the longitudinal direction of the guide wire 40 can be easily grasped. Therefore, the joint strength between the core shaft 9 and the coil body 23 can be easily adjusted.

According to the guide wire 40 of the present embodiment, since the intermediate connection portion 47 is colored, a length X1 of the intermediate connection portion 47 connected to the coil body 23 in the longitudinal direction of the guide wire 40 can be easily grasped. Therefore, the joint strength between the core shaft 9 and the coil body 23 can be easily adjusted.

### (Sixth Embodiment)

A sixth embodiment will be described.

A guide wire 50 of the sixth embodiment differs from the guide wire 30 of the fourth embodiment in the connection form between the coil body and the proximal end connection portion and the connection form between the coil body and the intermediate connection portion. That is, unlike the guide wire 30 of the fourth embodiment, the guide wire 50 of the present embodiment has the surface roughness of the coil body greater than the surface roughness of the core shaft.

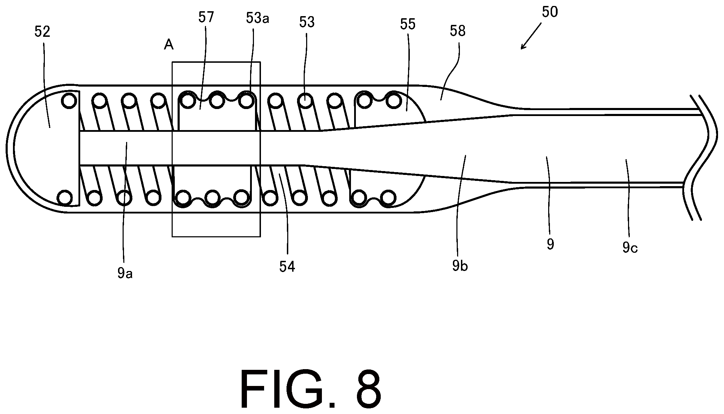

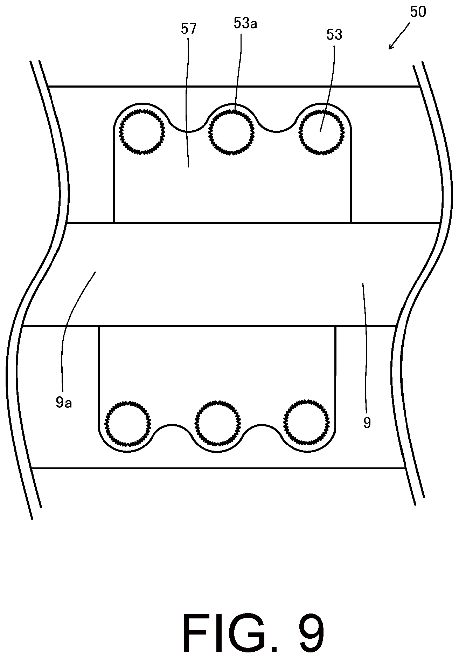

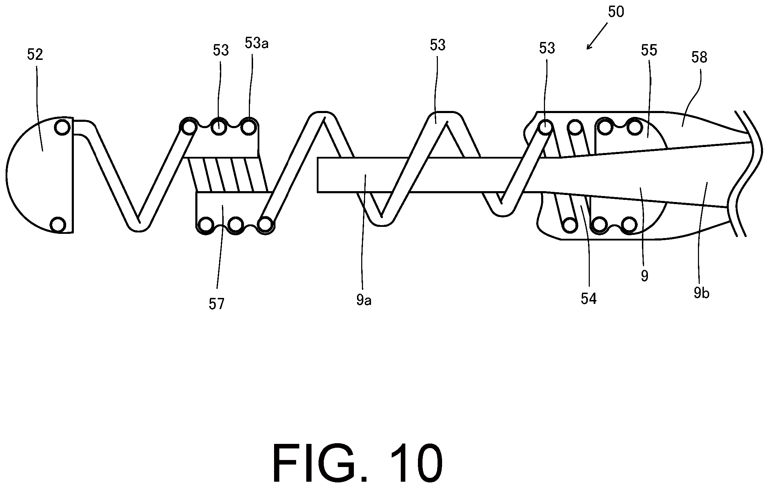

FIG. 8 is a longitudinal sectional view of the distal end of the guide wire of the sixth embodiment, FIG. 9 is an enlarged view of part A of FIG. 8, and FIG. 10 is an enlarged view of the distal end showing a state in which the coil body of the guide wire of the sixth embodiment is detached from the core shaft.

As shown in FIGS. 8 and 9, the guide wire 50 of the present embodiment includes a core shaft 9, a coil body 53 covering a distal end portion of the core shaft 9, a distal end connection portion 52 connected to a distal end of the coil body 53 and a distal end of the core shaft 9, a proximal end connection portion 55 connected to a proximal end of the coil body 53 and the core shaft 9, an intermediate connection portion 57 arranged between the distal end connection portion 52 and the proximal end connection portion 55 in the longitudinal direction of the guide wire 50 and connected to the coil body 53 and the core shaft 9, and a coating agent 58 covering outer peripheries of the core shaft 9, the coil body 53, the distal end connection portion 52, the proximal end connection portion 55, and the intermediate connection portion 57.

A gap 54 is formed between the core shaft 9 and the coil body 53.

The coil body 53 is a hollow cylindrical coil body formed by winding either of one or more metal strands or one or more resin strands. The coil body 53 is wound a plurality of times around the outer periphery of the core shaft 9. The distal end of the coil body 53 is connected to the distal end of the core shaft 9 by the distal end connection portion 52, and the proximal end of the coil body 53 is connected to the core shaft 9 by the proximal end connection portion 55.

The surface roughness of the strand constituting the coil body 53 is greater than the surface roughness of the core shaft 9. Convex/concave portions 53a are formed on the surface of the strand constituting the coil body 53 by processing means such as sandblasting.

The material of the strand forming the coil body 53 may be the same as that of the coil body 3 of the first embodiment. In the present embodiment, the material of the strand of the coil body 53 is stainless steel.

The distal end connection portion 52 joins the distal end of the coil body 53 and the distal end of the core shaft 9. The material of the distal end connection portion 52 may be the same as that of the distal end connection portion 2 of the first embodiment. In the present embodiment, the material of the distal end connection portion 52 is a silver-tin solder.

The proximal end connection portion 55 joins the proximal end of the coil body 53 and the core shaft 9. As shown in FIGS. 8 and 9, the outer diameter of the proximal end connection portion 55 is smaller than the outer diameter of the coil body 53 between the strands constituting the coil body 53.

The material of the proximal end connection portion 55 may be the same as that of the proximal end connection portion 5 of the first embodiment. In the present embodiment, the material of the proximal end connection portion 55 is an epoxy-based adhesive.

The intermediate connection portion 57 is arranged between the distal end connection portion 52 and the proximal end connection portion 55 in the longitudinal direction of the guide wire 50 and connects the coil body 53 and the core shaft 9. As shown in FIGS. 8 and 9, the outer diameter of the intermediate connection portion 47 is smaller than the outer diameter of the coil body 23 between the strands constituting the coil body 23.

The material of the intermediate connection portion 47 may be the same as those of the distal end side intermediate connection portion 7a and the proximal end side intermediate connection portion 7b of the second embodiment. In the present embodiment, the material of the intermediate connection portion 47 is an epoxy-based adhesive.

In the present embodiment as well, as shown in FIG. 8, one intermediate connection portion 57 is provided. The number of intermediate connection portions 57 is not limited to one, and may be two or more.

In the present embodiment, as shown in FIG. 8, the longitudinal width of the proximal end connection portion 55 is formed across two strands constituting the coil body 53, and one portion having an outer diameter smaller than the outer diameter of the coil body 53 is provided in the proximal end connection portion 55. The longitudinal width of the proximal end connection portion 55 may be formed across any number of strands of the coil body 53. A plurality of portions having an outer diameter smaller than the outer diameter of the coil body 53 may be formed in the proximal end connection portion 55.

The longitudinal width of the intermediate connection portion 57 is formed across three strands constituting the coil body 53, and two portions having an outer diameter smaller than the outer diameter of the coil body 53 are provided in the intermediate connection portion 57. The longitudinal width of the intermediate connection portion 57 may be formed across any number of strands of the coil body 53. The number of portions having an outer diameter smaller than the outer diameter of the coil body 53 may be one, or more than one in the intermediate connection portion 57.

The coating agent 58 covers the outer peripheries of the core shaft 9, the coil body 53, the distal end connection portion 52, the proximal end connection portion 55, and the intermediate connection portion 57. The material of the coating agent 58 may be the same as that of the coating agent 8 of the first embodiment. In the present embodiment, the material of the coating agent 58 is polyacrylamide.

In the guide wire 50 described above, if detachment of the coil body 53 from the core shaft 9 occurs during a procedure, the surface roughness of the strands constituting the coil body 53 is set to be greater than the surface roughness of the core shaft 9. Accordingly, as shown in FIG. 10, the intermediate connection portion 57 or the proximal end connection portion 55 is detached from the core shaft 9 integrally with the coil body 53, which can prevent at least one of the intermediate connection portion 57 itself and the proximal end connection portion 55 itself from being destroyed and flowing out into a lumen such as a patient's blood vessel.

The point that the coil body 53 and the core shaft 9 are connected by an adhesive made of a resin material with the surface roughness of the coil body 53 made greater than the surface roughness of the core shaft 9, as in the guide wire 50 of the present embodiment, can be applied to a connection structure in which a surface of a first member having a predetermined first surface roughness and a surface of a second member having a second surface roughness greater than the first surface roughness are connected by an adhesive made of a resin material.

In this case, even when a large force is applied to the connection structure and the second member is detached from the first member, the adhesive is detached in a state of being connected to the second member, which can prevent the adhesive itself from being crushed and scattered.

According to the guide wire 50 of the present embodiment, the guide wire 50 includes the core shaft 9, the coil body 53 covering the distal end portion of the core shaft 9, the distal end connection portion 52 connecting the distal end of the coil body 53 and the distal end of the core shaft 9, and the proximal end connection portion 55 connecting the proximal end of the coil body 53 and the core shaft 9, and since the proximal end connection portion 55 is composed of an adhesive made of a resin material, the guide wire 50 can be made flexible. Therefore, the insertability of the guide wire 50 into the patient's body can be improved.

According to the guide wire 50 of the present embodiment, the intermediate connection portion 57 connecting the coil body 53 and the core shaft 9 is provided between the distal end connection portion 52 and the proximal end connection portion 55, and since the intermediate connection portion 57 is composed of an adhesive made of a resin material, the guide wire 50 can be made flexible. Therefore, the insertability of the guide wire 50 into the patient's body can be improved.

According to the guide wire 50 of the present embodiment, the coil body 53 is wound a plurality of times around the outer periphery of the core shaft 9, and since the proximal end connection portion 55 is formed to have an outer diameter smaller than the outer diameter of the coil body 53 between adjacent strands constituting the coil body 23, the guide wire 50 can be made even more flexible. Therefore, the insertability of the guide wire 50 into the patient's body can be further improved.

According to the guide wire 50 of the present embodiment, the coil body 53 is wound a plurality of times around the outer periphery of the core shaft 9, and since the intermediate connection portion 57 is formed to have an outer diameter smaller than the outer diameter of the coil body 53 between adjacent strands constituting the coil body 53, the guide wire 50 can be made even more flexible. Therefore, the insertability of the guide wire 50 into the patient's body can be further improved.

According to the guide wire 50 of the present embodiment, since the surface roughness of the coil body 53 is greater than the surface roughness of the core shaft 9, even in a case where a large force is applied to the guide wire 50 and the coil body 53 is detached from the core shaft 9, the proximal end connection portion 55 and the intermediate connection portion 57 are detached in a state of being connected to the coil body 53, so that at least one of the proximal end connection portion 55 itself and the intermediate connection portion 57 itself can be prevented from being crushed and scattered.

### (Seventh Embodiment)

A seventh embodiment will be described.

A guide wire 60 of the seventh embodiment differs from the guide wire 50 of the sixth embodiment in the connection form between the coil body and the proximal end connection portion and the connection form between the coil body and the intermediate connection portion. That is, the guide wire 50 of the sixth embodiment was configured such that the proximal end connection portion 55 and the intermediate connection portion 57 were detached in a state of being connected to the coil body 53 by making the surface roughness of the coil body greater than the surface roughness of the core shaft. The guide wire 60 of the present embodiment is configured such that the proximal end connection portion and the intermediate connection portion are detached in a state of being connected to the coil body by providing an oxide film on the surface of the coil body and not providing an oxide film on the surface of the core shaft.

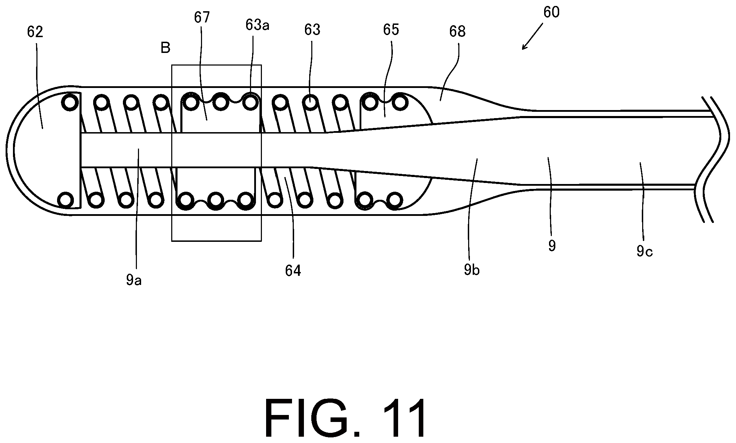

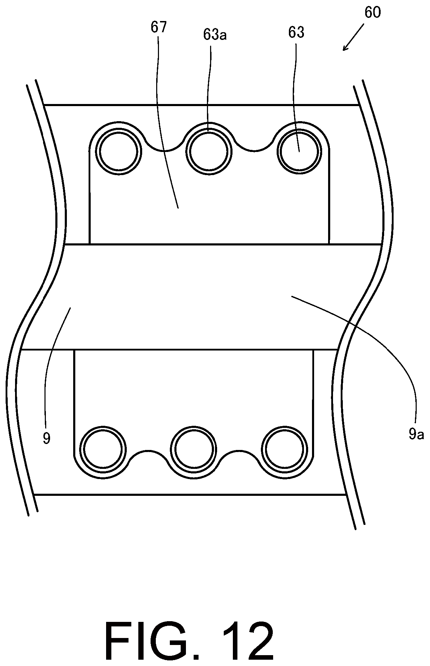

FIG. 11 is a longitudinal sectional view of a distal end of the guide wire of the seventh embodiment, and FIG. 12 is an enlarged view of part B of FIG. 11.

The guide wire 60 of the present embodiment includes a core shaft 9, a coil body 63 covering a distal end portion of the core shaft 9, a distal end connection portion 62 connected to a distal end of the coil body 63 and a distal end of the core shaft 9, a proximal end connection portion 65 connected to a proximal end of the coil body 63 and the core shaft 9, an intermediate connection portion 67 arranged between the distal end connection portion 62 and the proximal end connection portion 65 in the longitudinal direction of the guide wire 60 and connected to the coil body 63 and the core shaft 9, and a coating agent 68 covering outer peripheries of the core shaft 9, the coil body 63, the distal end connection portion 62, the proximal end connection portion 65, and the intermediate connection portion 67.

A gap 64 is formed between the core shaft 9 and the coil body 63.

The coil body 63 is a hollow cylindrical coil body formed by winding either of one or more metal strands or one or more resin strands. The coil body 63 is wound a plurality of times around the outer periphery of the core shaft 9. The distal end of the coil body 63 is connected to the distal end of the core shaft 9 by the distal end connection portion 62, and the proximal end of the coil body 63 is connected to the core shaft 9 by the proximal end connection portion 65.

The material of the strand forming the coil body 63 may be the same as that of the coil body 3 of the first embodiment. In the present embodiment, the material of the strand forming the coil body 63 is stainless steel. On the surface of the strand constituting the coil body 63, an oxide film 63a is formed in which oxygen is combined mainly with chromium in the stainless steel by oxidation.

The distal end connection portion 62 joins the distal end of the coil body 63 and the distal end of the core shaft 9. The material of the distal end connection portion 62 may be the same as that of the distal end connection portion 2 of the first embodiment. In the present embodiment, the material of the distal end connection portion 62 is a silver-tin solder.

The proximal end connection portion 65 joins the proximal end of the coil body 63 and the core shaft 9. The outer diameter of the proximal end connection portion 65 is smaller than the outer diameter of the coil body 63 between the strands constituting the coil body 63.

The material of the proximal end connection portion 65 may be the same as that of the proximal end connection portion 5 of the first embodiment. In the present embodiment, the material of the proximal end connection portion 65 is an epoxy-based adhesive.

The intermediate connection portion 67 is arranged between the distal end connection portion 62 and the proximal end connection portion 65 in the longitudinal direction of the guide wire 60 and connects the coil body 63 and the core shaft 9. The outer diameter of the intermediate connection portion 67 is smaller than the outer diameter of the coil body 63 between the strands constituting the coil body 63.

The material of the intermediate connection portion 67 may be the same as those of the distal end side intermediate connection portion 7a and the proximal end side intermediate connection portion 7b of the second embodiment. In the present embodiment, the material of the intermediate connection portion 67 is an epoxy-based adhesive.

In the present embodiment, one intermediate connection portion 67 is provided. The number of intermediate connection portions 67 is not limited to one, and may be two or more.

In the present embodiment, the longitudinal width of the proximal end connection portion 65 is formed across two strands constituting the coil body 63, and one portion having an outer diameter smaller than the outer diameter of the coil body 63 is provided in the proximal end connection portion 65. The longitudinal width of the proximal end connection portion 65 may be formed across any number of strands of the coil body 63. A plurality of portions having an outer diameter smaller than the outer diameter of the coil body 63 may be formed in the proximal end connection portion 65.

The longitudinal width of the intermediate connection portion 67 is formed across three strands constituting the coil body 63, and two portions having an outer diameter smaller than the outer diameter of the coil body 63 are provided in the intermediate connection portion 67. The longitudinal width of the intermediate connection portion 67 may be formed across any number of strands of the coil body 63. The number of portions having an outer diameter smaller than the outer diameter of the coil body 63 may be one, or three or more in the intermediate connection portion 67.

The coating agent 68 covers the outer peripheries of the core shaft 9, the coil body 63, the distal end connection portion 62, the proximal end connection portion 65, and the intermediate connection portion 67. The material of the coating agent 68 may be the same as that of the coating agent 8 of the first embodiment. In the present embodiment, the material of the coating agent 68 is polyacrylamide.

In the guide wire 60 described above, if the coil body 63 is detached from the core shaft 9 during a procedure, by the strands constituting the coil body 63 having the oxide film 63a formed on the surfaces thereof, the intermediate connection portion 67 and the proximal end connection portion 65 are detached from the core shaft 9 integrally with the coil body 63, which can prevent either the intermediate connection portion 67 itself or the proximal end connection portion 65 itself from being destroyed and flowing out into a lumen such as a patient's blood vessel.

The point that the surface of the core shaft 9 on which no oxide film is formed and the surface of the coil body 63 on which an oxide film is formed are connected by an adhesive made of a resin material, as in the guide wire 60 of the present embodiment, can be applied to a connection structure in which a surface of a first member on which no oxide film is formed and a surface of a second member on which an oxide film is formed are connected by an adhesive made of a resin material.

In this case, even when a large force is applied to the connection structure and the second member is detached from the first member, the adhesive is detached in a state of being connected to the second member, which can prevent the adhesive itself from being crushed and scattered.

According to the guide wire 60 of the present embodiment, the guide wire 60 includes the core shaft 9, the coil body 63 covering the distal end portion of the core shaft 9, the distal end connection portion 62 connecting the distal end of the coil body 63 and the distal end of the core shaft 9, and the proximal end connection portion 65 connecting the proximal end of the coil body 63 and the core shaft 9, and since the proximal end connection portion 65 is composed of an adhesive made of a resin material, the guide wire 60 can be made flexible. Therefore, the insertability of the guide wire 60 into the patient's body can be improved.

According to the guide wire 60 of the present embodiment, the intermediate connection portion 67 connecting the coil body 63 and the core shaft 9 is provided between the distal end connection portion 62 and the proximal end connection portion 65, and since the intermediate connection portion 67 is composed of an adhesive made of a resin material, the guide wire 60 can be made flexible. Therefore, the insertability of the guide wire 60 into the patient's body can be improved.

According to the guide wire 60 of the present embodiment, the coil body 63 is wound a plurality of times around the outer periphery of the core shaft 9, and since the proximal end connection portion 65 is formed at a position lower than the outer diameter of the coil body 63 between adjacent strands constituting the coil body 63, the guide wire 60 can be made even more flexible. Therefore, the insertability of the guide wire 60 into the patient's body can be further improved.

According to the guide wire 60 of the present embodiment, the coil body 63 is wound a plurality of times around the outer periphery of the core shaft 9, and since the intermediate connection portion 67 is formed to have an outer diameter smaller than the outer diameter of the coil body 63 between adjacent strands constituting the coil body 63, the guide wire 60 can be made even more flexible. Therefore, the insertability of the guide wire 60 into the patient's body can be further improved.

According to the guide wire 60 of the present embodiment, since the oxide film 63a is formed on the surface of the coil body 63, even in a case where a large force is applied to the guide wire 60 and the coil body 63 is detached from the core shaft 9, the proximal end connection portion 65 and the intermediate connection portion 67 are detached in a state of being connected to the coil body 63, so that at least one of the proximal end connection portion 65 itself and the intermediate connection portion 67 itself can be prevented from being crushed and scattered.

### (Eighth Embodiment)

An eighth embodiment of the present disclosure will be described.

A guide wire 70 of the eighth embodiment has an intermediate connection portion as compared with the guide wire 1 of the first embodiment, and differs in the connection form between the coil body and the proximal end connection portion and the connection form between the coil body and the intermediate connection portion. That is, the guide wire 70 of the eighth embodiment is configured such that the proximal end connection portion and the intermediate connection portion are detached in a state of being connected to the coil body by forming a plurality of grooves on the surface of the coil body in a direction intersecting the longitudinal direction of the core shaft.

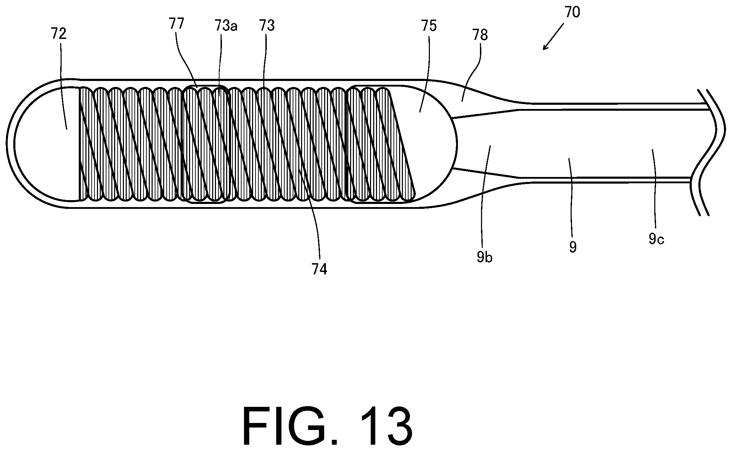

FIG. 13 is an enlarged view of a distal end of the guide wire of the eighth embodiment.

The guide wire 70 of the present embodiment includes a core shaft 9, a coil body 73 covering a distal end portion of the core shaft 9, a distal end connection portion 72 connected to a distal end of the coil body 73 and a distal end of the core shaft 9, a proximal end connection portion 75 connected to a proximal end of the coil body 73 and the core shaft 9, an intermediate connection portion 77 arranged between the distal end connection portion 72 and the proximal end connection portion 75 in the longitudinal direction of the guide wire 70 and connected to the coil body 73 and the core shaft 9, and a coating agent 78 covering outer peripheries of the core shaft 9, the coil body 73, the distal end connection portion 72, the proximal end connection portion 75, and the intermediate connection portion 77.

Inside the coil body 73, a gap 74 is formed between the core shaft 9 and the coil body 73.

The coil body 73 is a hollow cylindrical coil body formed by winding either of one or more metal strands or one or more resin strands. The coil body 73 is wound a plurality of times around the outer periphery of the core shaft 9. The distal end of the coil body 73 is connected to the distal end of the core shaft 9 by the distal end connection portion 72, and the proximal end of the coil body 73 is connected to the core shaft 9 by the proximal end connection portion 75.

The material of the strand forming the coil body 73 may be the same as that of the coil body 3 of the first embodiment. In the present embodiment, the material of the strand forming the coil body 73 is stainless steel. On the surface of the strand constituting the coil body 73, a plurality of grooves 73a are formed in a direction substantially vertical to the longitudinal direction of the core shaft 9. In FIG. 13, the plurality of grooves 73a are indicated by vertical-stripe hatching.

In the present embodiment, a form in which the plurality of grooves 73a are formed in a direction substantially vertical to the longitudinal direction of the core shaft 9 on the surface of the strand constituting the coil body 73 is described. These plurality of grooves are not limited to those formed in a direction substantially vertical to the longitudinal direction of the core shaft, and may be any grooves formed on the surface of the strand constituting the coil body in a direction intersecting the longitudinal direction of the core shaft.

The distal end connection portion 72 joins the distal end of the coil body 73 and the distal end of the core shaft 9. The material of the distal end connection portion 72 may be the same as that of the distal end connection portion 2 of the first embodiment. In the present embodiment, the material of the distal end connection portion 72 is a silver-tin solder.

The proximal end connection portion 75 joins the proximal end of the coil body 73 and the core shaft 9. The material of the proximal end connection portion 75 may be the same as that of the proximal end connection portion 5 of the first embodiment. In the present embodiment, the material of the proximal end connection portion 75 is an epoxy-based adhesive.

The intermediate connection portion 77 is arranged between the distal end connection portion 72 and the proximal end connection portion 75 in the longitudinal direction of the guide wire 70 and connects the coil body 73 and the core shaft 9. The material of the intermediate connection portion 77 may be the same as those of the distal end side intermediate connection portion 7a and the proximal end side intermediate connection portion 7b of the second embodiment. In the present embodiment, the material of the intermediate connection portion 77 is an epoxy-based adhesive.

In the present embodiment as well, as shown in FIG. 13, one intermediate connection portion 77 is provided. The number of intermediate connection portions 77 is not limited to one, and may be two or more.

The longitudinal width of the proximal end connection portion 75 may be formed across any number of strands of the coil body 73. The longitudinal width of the intermediate connection portion 77 may be formed across any number of strands of the coil body 73.

The coating agent 78 covers the outer peripheries of the core shaft 9, the coil body 73, the distal end connection portion 72, the proximal end connection portion 75, and the intermediate connection portion 77. The material of the coating agent 78 may be the same as that of the coating agent 8 of the first embodiment. In the present embodiment, the material of the coating agent 78 is polyacrylamide.

In the guide wire 70 described above, if detachment of the coil body 73 from the core shaft 9 occurs during a procedure, by the coil body 73 composed of strands having the plurality of grooves 73a formed in a direction substantially vertical to the longitudinal direction of the core shaft 9, the intermediate connection portion 77 and the proximal end connection portion 75 are detached from the core shaft 9 integrally with the coil body 73, which can prevent at least one of the intermediate connection portion 77 itself and the proximal end connection portion 75 itself from being destroyed and flowing out into a lumen such as a patient's blood vessel.

According to the guide wire 70 of the present embodiment, the guide wire 70 includes the core shaft 9, the coil body 73 covering the distal end portion of the core shaft 9, the distal end connection portion 72 connecting the distal end of the coil body 73 and the distal end of the core shaft 9, and the proximal end connection portion 75 connecting the proximal end of the coil body 73 and the core shaft 9, and since the proximal end connection portion 75 is composed of an adhesive made of a resin material, the guide wire 70 can be made flexible. Therefore, the insertability of the guide wire 70 into the patient's body can be improved.

According to the guide wire 70 of the present embodiment, the intermediate connection portion 77 connecting the coil body 73 and the core shaft 9 is provided between the distal end connection portion 72 and the proximal end connection portion 75, and since the intermediate connection portion 77 is composed of an adhesive made of a resin material, the guide wire 70 can be made flexible. Therefore, the insertability of the guide wire 70 into the patient's body can be improved.

According to the guide wire 70 of the present embodiment, since the plurality of grooves 73a are formed on the surface of the coil body 73 in a direction intersecting the longitudinal direction of the core shaft 9, even in a case where a large force is applied to the guide wire 70 and the coil body 73 is detached from the core shaft 9, the proximal end connection portion 75 and the intermediate connection portion 77 are detached in a state of being connected to the coil body 73, so that at least one of the proximal end connection portion 75 itself and the intermediate connection portion 77 itself can be prevented from being crushed and scattered.

### (Ninth Embodiment)

A ninth embodiment of the present disclosure will be described.

A guide wire 70 of the ninth embodiment differs from the guide wire 30 of the fourth embodiment in the configuration of the core shaft. That is, a core shaft 81 of the ninth embodiment has a plurality of grooves 82 formed in a direction intersecting the longitudinal direction of the core shaft 81, and is configured such that the proximal end connection portion and the intermediate connection portion are detached in a state of being connected to the coil body.

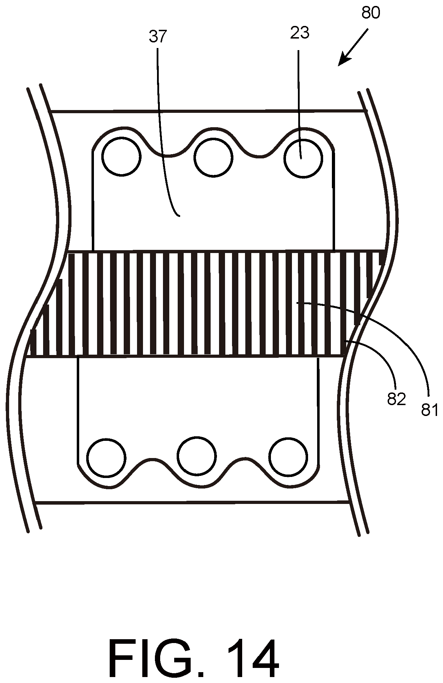

FIG. 14 is a partially enlarged view of the guide wire of the ninth embodiment. FIG. 14 is an enlarged view of a portion corresponding to the intermediate connection portion 37 of the guide wire 30 of the fourth embodiment in the guide wire according to the ninth embodiment. FIG. 14 shows a cross section of the intermediate connection portion 37 and a side surface of the core shaft 81.

The core shaft 81 has a plurality of grooves 82 in a direction intersecting the longitudinal direction of the core shaft 81. The grooves 82 may be provided over the entire core shaft 81, or may be provided only in a range connected to either the intermediate connection portion 37 or the proximal end connection portion 35.

According to the guide wire 80 of the present embodiment, since the plurality of grooves 82 are formed on the surface of the core shaft 81 in a direction intersecting the longitudinal direction of the core shaft 81, even in a case where a large force is applied to the guide wire 80 and the coil body 23 is detached from the core shaft 81, the proximal end connection portion 35 and the intermediate connection portion 37 are detached in a state of being connected to the coil body 23, so that at least one of the proximal end connection portion 35 itself and the intermediate connection portion 37 itself can be prevented from being crushed and scattered.

### (Tenth Embodiment)

A tenth embodiment of the present disclosure will be described.

A guide wire 90 of the tenth embodiment differs from the guide wire 30 of the fourth embodiment in the configuration of the core shaft. That is, a core shaft 91 of the tenth embodiment has a plurality of grooves 92 formed along the longitudinal direction of the core shaft 91, and is configured such that the coil body 23 is detached from the core shaft 91 in a state in which the proximal end connection portion and the intermediate connection portion are connected to the core shaft 91.

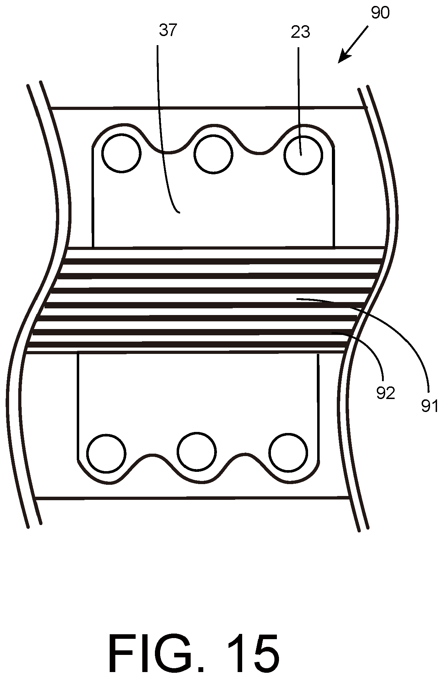

FIG. 15 is a partially enlarged view of the guide wire of the tenth embodiment. FIG. 15 is an enlarged view of a portion corresponding to the intermediate connection portion 37 of the guide wire 30 of the fourth embodiment in the guide wire according to the tenth embodiment. FIG. 15 shows a cross section of the intermediate connection portion 37 and a side surface of the core shaft 91.

The core shaft 91 has a plurality of grooves 92 along the longitudinal direction of the core shaft 91. The grooves 92 may be provided over the entire core shaft 91, or may be provided only in a range connected to either the intermediate connection portion 37 or the proximal end connection portion 35.

According to the guide wire 90 of the present embodiment, since the plurality of grooves 92 are formed on the surface of the core shaft 91 in a direction intersecting the longitudinal direction of the core shaft 91, even in a case where a large force is applied to the guide wire 90 and the coil body 23 is detached from the core shaft 91, the proximal end connection portion 35 and the intermediate connection portion 37 maintain a state of being connected to the core shaft 91, so that at least one of the proximal end connection portion 35 itself and the intermediate connection portion 37 itself can be prevented from being crushed and scattered.

While the guide wires of several embodiments of the present disclosure have been described above. The present disclosure is not limited to the above embodiments, and various modifications can be made without departing from the gist thereof.

For example, the point that either the proximal end connection portion or the intermediate connection portion is colored as in the guide wire 40 of the fifth embodiment may be applied to: the distal end connection portion 2 and the proximal end connection portion 5 of the guide wire 1 of the first embodiment; the distal end connection portion 2, the distal end side intermediate connection portion 7a, the proximal end side intermediate connection portion 7b, and the proximal end connection portion 5 of the guide wire 10 of the second embodiment; the distal end connection portion 22 and the proximal end connection portion 25 of the guide wire 20 of the third embodiment; the distal end connection portion 22 of the guide wire 40 of the fifth embodiment; the distal end connection portion 52, the intermediate connection portion 57, and the proximal end connection portion 55 of the guide wire 50 of the sixth embodiment; the distal end connection portion 62, the intermediate connection portion 67, and the proximal end connection portion 65 of the guide wire 60 of the seventh embodiment; and the distal end connection portion 72, the intermediate connection portion 77, and the proximal end connection portion 75 of the guide wire 70 of the eighth embodiment.

In this case, each of the length of the distal end connection portion, the length of the intermediate connection portion, and the length of the proximal end connection portion can be easily grasped, and the joint strength between each core shaft and the coil body can be easily adjusted.

The point that the outer diameters of the intermediate connection portion and the proximal end connection portion are made smaller than the outer diameter of the coil body between the strands constituting the coil body may be applied to the distal end connection portion 72, the intermediate connection portion 77, and the proximal end connection portion 75 of the guide wire 70 of the eighth embodiment.

In this case, the guide wire 70 can be made even more flexible. Therefore, the insertability of the guide wire 70 into the patient's body can be further improved.

The following points may be applied to the guide wire 1 of the first embodiment, the guide wire 10 of the second embodiment, and the guide wire 20 of the third embodiment: the point that the surface roughness of the coil body is made greater than the surface roughness of the core shaft as in the guide wire 50 of the sixth embodiment; the point that an oxide film is formed on the surface of the coil body and no oxide film is formed on the core shaft as in the guide wire 60 of the seventh embodiment; and the point that a plurality of grooves are formed on the surface of the coil body in a direction intersecting the longitudinal direction of the core shaft as in the guide wire 70 of the eighth embodiment.

In this case, even in a case where a large force is applied to the guide wire and the coil body is detached from the core shaft, the proximal end connection portion and the intermediate connection portion are detached in a state of being connected to the coil body, so that at least one of the proximal end connection portion itself and the intermediate connection portion itself can be prevented from being crushed and scattered.

The point that the plurality of grooves 82 are formed on the surface of the core shaft 81 in a direction intersecting the longitudinal direction of the core shaft 81 as in the guide wire 80 of the ninth embodiment may be applied to the core shafts of the guide wires of the first to third embodiments and the fifth to eighth embodiments.

In this case, even in a case where a large force is applied to the guide wire and the coil body is detached from the core shaft, the proximal end connection portion and the intermediate connection portion are detached in a state of being connected to the coil body, so that at least one of the proximal end connection portion itself and the intermediate connection portion itself can be prevented from being crushed and scattered.

The point that the plurality of grooves 92 are formed on the surface of the core shaft 91 along the longitudinal direction of the core shaft 91 as in the guide wire 90 of the tenth embodiment may be applied to the core shafts of the guide wires of the first to third embodiments and the fifth embodiment.

In this case, even in a case where a large force is applied to the guide wire and the coil body is detached from the core shaft, the proximal end connection portion 35 and the intermediate connection portion 37 maintain a state of being connected to the core shaft 91, so that at least one of the proximal end connection portion 35 itself and the intermediate connection portion 37 itself can be prevented from being crushed and scattered.

## Claims

1. A guide wire (1, 10, 20, 30, 40, 50, 60, 70, 80, 90) comprising:
a core shaft (9, 81, 91);
a coil body (3, 23, 53, 63) covering a distal end portion of the core shaft;
a distal end connection portion (2, 22, 52, 62, 72) connecting a distal end of the coil body and a distal end of the core shaft; and
a proximal end connection portion (5, 25, 35, 45, 55, 65, 75) connecting a proximal end of the coil body and the core shaft and containing an adhesive made of a resin material.

2. The guide wire (20, 30, 40, 50, 60, 80, 90) according to claim 1, wherein
the coil body (23, 53, 63) is wound a plurality of times around an outer periphery of the core shaft, and
the proximal end connection portion (25, 35, 45, 55, 65) is formed to have an outer diameter smaller than an outer diameter of the coil body between adjacent strands of the coil body.

3. The guide wire (40) according to claim 1 or 2, wherein the proximal end connection portion (45) is colored.

4. The guide wire (30, 40, 50, 60) according to any one of claims 1 to 3, comprising an intermediate connection portion (7, 7a, 7b, 37, 47, 57, 67) connecting the coil body and the core shaft between the distal end connection portion and the proximal end connection portion,
wherein the intermediate connection portion contains an adhesive made of a resin material.

5. The guide wire (30, 40, 50, 60) according to claim 4, wherein
the coil body (23) is wound a plurality of times around an outer periphery of the core shaft, and
the intermediate connection portion (37, 47, 57, 67) is formed to have an outer diameter smaller than the outer diameter of the coil body between adjacent strands of the coil body.

6. The guide wire (40) according to claim 4 or 5, wherein the intermediate connection portion (47) is colored.

7. The guide wire (50) according to any one of claims 1 to 6, wherein a surface roughness of the coil body (53) is greater than a surface roughness of the core shaft (9).

8. The guide wire (60) according to any one of claims 1 to 7, wherein an oxide film (63a) is formed on a surface of the coil body (63).

9. The guide wire (70) according to any one of claims 1 to 8, wherein a plurality of grooves (73a) are formed on a surface of the coil body (73) in a direction intersecting a longitudinal direction of the core shaft (9).

10. The guide wire (80) according to any one of claims 1 to 9, wherein a plurality of grooves (82) are formed on a surface of the core shaft (81) in a direction intersecting a longitudinal direction of the core shaft (81).

11. The guide wire (90) according to any one of claims 1 to 9, wherein a plurality of grooves (92) are formed on a surface of the core shaft (91) along a longitudinal direction of the core shaft (91).

12. A connection structure in which a surface of a first member (53) having a first surface roughness and a surface of a second member (9) having a second surface roughness greater than the first surface roughness are connected by an adhesive made of a resin material.

13. A connection structure in which a surface of a first member (9) on which no oxide film is formed and a surface of a second member (63) on which an oxide film (63a) is formed are connected by an adhesive made of a resin material.
